(19) Europäisches Patentamt

European Patent Office

Office européen des brevets

(11) Veröffentlichungsnummer: **0 266 689 B1**

(12) **EUROPÄISCHE PATENTSCHRIFT**

(45) Veröffentlichungstag der Patentschrift: **02.01.92**

(21) Anmeldenummer: **87115957.0**

(22) Anmeldetag: **30.10.87**

(51) Int. Cl.⁵: **C07C 67/333**, C07C 69/533

(54) Verfahren zur Herstellung von 4-Pentensäureestern.

(30) Priorität: **07.11.86 DE 3638011**

(43) Veröffentlichungstag der Anmeldung:
**11.05.88 Patentblatt 88/19**

(45) Bekanntmachung des Hinweises auf die
Patenterteilung:
**02.01.92 Patentblatt 92/01**

(84) Benannte Vertragsstaaten:
**BE CH DE ES FR GB IT LI NL**

(56) Entgegenhaltungen:
**EP-A- 0 126 349**
**DE-A- 3 109 312**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**
Erfinder: **Aichinger, Heinrich, Dr.**
**Rathenaustrasse 6**
**W-6800 Mannheim 1(DE)**
Erfinder: **Naeumann, Fritz, Dr.**
**Rathenaustrasse 3 a**
**W-6800 Mannheim 1(DE)**
Erfinder: **Fischer, Rolf, Dr.**
**Bergstrasse 98**
**W-6900 Heidelberg(DE)**

**Beschreibung**

Bei der Herstellung von Pentensäureestern durch Umsetzung von Butadien mit Kohlenmonoxid und Alkoholen in Gegenwart von Metallcarbonylkatalysatoren, wie es z.B. in der DE-OS 30 40 432 beschrieben wird, erhält man erhebliche Mengen an isomeren Pentensäureestern. Für weitere Umsetzungen, z.B. zum herstellen von 5-Formylvaleriansäureestern durch Hydroformylierung von Pentensäureestern wird jedoch 4-Pentensäureestern zu erhalten. Wie aus Bull. of the Chem. Soc. of Japan, Band 46, Seite 548, bekannt ist, erhält man durch Isomerisieren von 3-Pentensäuremethylester in Gegenwart von Cobaltcarbonylen jedoch vorwiegend 2-Pentensäuremethylester. Nach einer anderen in Tetrahedron Vol. 28, Seiten 5769-77 (1972) beschriebenen Arbeitsweise gelingt es zwar in Gegenwart eines Komplexes aus Rhodiumtriphenylphosphin und Zinnchlorid das Isomerengleichgewicht so zu verschieben, daß 4-Pentensäureester erhalten werden. Der dort verwendete Katalysator wird jedoch innerhalb weniger Stunden inaktiv.

In der EP-Anmeldung 226 349 wird ein Verfahren zur Herstellung von 4-Pentensäureester aus einem Pentensäureestergemisch durch Behandeln mit sauren Zeolithen, die einen Gehalt an Palladium, Ruthenium oder Rhodium haben, beschrieben. Die Lebensdauer der Katalysatoren ist jedoch noch verbesserungsbedürftig.

Bei der Isomerisierung von Pentensäureestern liegen nach Erreichen des thermodynamischen Gleichgewichts fünf Isomere, nämlich 4-Pentensäureester, cis- und trans-3-Pentensäureester sowie cis- und trans-2-Pentensäureester vor, wobei das Gleichgewicht stark nach der Seite des trans-2-Pentensäureesters verschoben ist.

Es war deshalb die technische Aufgabe gestellt, ein Verfahren zur Herstellung von 4-Pentensäureestern aus isomeren Pentensäureestern zur Verfügung zu stellen, bei dem der verwendete Katalysator eine lange Lebensdauer hat und leicht regenerierbar ist und zudem die lineare Verschiebung der Doppelbindung zum 4-Pentensäureester bevorzugt verläuft und zudem möglichst wenig des schwer abtrennbaren cis-2-Pentensäureesters gebildet wird.

Diese technische Aufgabe wird gelöst in einem Verfahren zur Herstellung von 4-Pentensäureestern durch Isomerisierung, wobei man isomere Pentensäureester bei erhöhter Temperatur mit Zeolithen behandelt und 4-Pentensäureester aus dem Reaktionsgemisch abdestilliert, dadurch gekennzeichnet, daß man Pentensäureester im Gemisch mit Alkanolen einsetzt.

Das neue Verfahren hat den Vorteil, daß durch den Alkoholzusatz zu den Pentensäureestern die Standzeit der Zeolith-Katalysatoren erhöht wird.

Vorteilhaft geht man von isomeren Pentensäureestern, z.B. 2- oder 3-Pentensäureestern aus, die sich von Alkanolen mit bis zu 12 Kohlenstoffatomen herleiten. Besonders bevorzugt verwendet man isomere Pentensäurealkylester insbesondere von Alkanolen mit bis zu 4 Kohlenstoffatomen. Geeignete Ausgangsstoffe sind beispielsweise 3-Pentensäuremethylester, 3-Pentensäureethylester, 3-Pentensäureethylester sowie 2-Pentensäurepropylester. Geeignet sind auch Gemisch aus isomeren Pentensäureestern, wie sie bei der Umsetzung von Butadien mit Kohlenmonoxid und Alkoholen in Gegenwart von Metallcarbonylen entsprechend der DE-OS 30 40 432 erhalten werden. Besonders bevorzugt sind 3-Pentensäureester.

Die Isomerisierung wird unter Mitverwendung von Alkanolen insbesondere mit 1 bis 6 Kohlenstoffatomen durchgeführt. Geeignete Alkanole sind beispielsweise Methanol, Ethanol, Propanol, Butanol, Isopropanol oder Isobutanol, sekundäre butanole, n-Pentanol oder n-Hexanol. Besonders geeignet sind Alkanole mit bis zu 3 Kohlenstoffatomen, z.B. Methanol, Ethanol und Propanole.

Als Katalysatoren für das erfindungsgemäße Verfahren werden acide zeolithische Katalysatoren eingesetzt. Zeolithe sind kristalline Aluminosilikate, die eine hochgeordnete Struktur mit einem Starren dreidimensionalen Netzwerk von $SiO_4$- und $AlO_4$-Tetradern besitzen, die durch gemeinsame Sauerstoffatome verbunden sind. Das Verhältnis der Si-und Al-Atome zu Sauerstoff beträgt 1 : 2 (siehe Ullmanns Encyclopädie d. techn. Chemie, 4. Auflage, Band 24, Seite 575 (1983). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen konnen anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt (siehe Ullmanns Encyclopädie d. techn. Chemie, 4. Aufl., Bd. 24, S. 575 (1983). So bilden bei der Mordernit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind vorzugsweise Zeolithe aus der Mordernit-Gruppe oder engporige Zeolithe vom Erionit-bzw. Chabasit-Typ oder Zeolithe vom Faujasit-Typ, z.B. Y-, X- oder L-Zeolithe. In diese Gruppe von Zeolithen gehören auch die sogenannten "ultrastabilen" Zeolithe des Faujasit-Typs, d.h. dealuminierte Zeolithe. Verfahren zur Herstellung solcher Zeolithe sind beschrieben in "Catalysis by Zeolites" Band 5 aus "Studies in Surface Science and Catalysis" ed. B. Imelik et. a. Elsevier Scientific Publishing Comp. 1980, S. 203 und "Crystal Structures of Ultra-stable Faujasites" Advances in Chemistry Series Nr. 101, American Chemical Society Washington, DC, S. 226 ff (1971) und in US-PS 4 512 961.

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen (vgl. Ullmanns Encyclopädie d. techn. Chem., 4. Aufl., Bd. 24, Seite 575 (1983).

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro-, Gallium- und Eisengermanatzeolithe oder deren Gemische.

Insbesondere eignen sich die Alumino-, Boro- und Eisensilikatzeolithe des Pentasiltyps für das erfindungsgemäße Verfahren. Der Aluminiumsilikatzeolith wird z.B. aus einer Aluminiumverbindung, vorzugsweise Al(OH)$_3$ oder Al$_2$(SO$_4$)$_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin-oder Triethylentetramin-Lösung mit oder insbesondere ohne Alkali- oder Erdalkalizusatz bei 100 bis 220 °C unter autogenem Druck hergestellt. Auch gehören hierzu die isotaktischen Zeolithe nach DE-OS 3 006 471. Die erhaltenen Aluminosilikatzeolithe enthalten je nach Wahl der Einsatzstoffmengen ein $SiO_2/Al_2O_3$-Verhältnis von 10 bis 40 000. Auch lassen sich derartige Aluminosilikatzeolithe in etherischem Medium wie Diethylenglykoldimethylether, in alkoholischem Medium wie Methanol bzw. 1,4-Butandiol oder in Wasser snythetisieren.

Der Borosilikatzeolith wird z.B. bei 90 bis 200 °C unter autogenem Druck synthetisiert, indem man eine Borverbindung, z.B. H$_3$BO$_3$, mit einer Siliciumverbindung, vorzugsweise hochdispersen Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch gehören hierzu die isotaktischen Zeolithe nach DE-OS 3 006 471 und EP 46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol, durchführt.

Den Eisensilikatzeolith erhält man z.B. aus einer Eisenverbindung, vorzugsweise Fe$_2$(SO$_4$)$_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220 °C unter autogenem Druck.

Zu den verwendeten siliciumreichen Zeolithen ($SiO_2/Al_2O_3$ größer oder gleich 10) gehören auch die bekannten ZSM-Typen sowie Ferrierit und NU-1 sowie Silicalite®, ein Molekularsieb, ein sog. Silica Polymorph.

Die so hergestellten Alumino-, Boro- und Eisensilikatzeolithe können nach ihrer Isolierung, Trocknung bei 100 bis 160 °C, vorzugsweise 110 °C, und Calcinierung bei 450 bis 550 °C, vorzugsweise 500 °C, mit einem Bindemittel im Verhältnis 90 : 10 bis 40 : 60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 h getrocknet und bei 500 °C/16h calciniert.

Man erhält auch vorteilhafte Katalysatoren, wenn der isolierte Alumino-bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester oder Graphit oder deren Gemische verwendet werden.

Liegt der Zeolith aufgrund der Art seiner Herstellung nicht in der vorteilhaft katalytisch aktiven, aciden H-Form vor, sondern z.B. in der Na-Form, dann dann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abtrennen der Koksablagerung mit Luft oder mit einem Luft/$N_2$-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, ist es vorteilhaft, die Zeolithe zu modifizieren. Eine geeignete Modifizierung der katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4. - 8. Nebengruppe wie Ti, Zr, V, Nb, Cr, Mo, W, Mn Re, Fe, Ru, Os, Co, Rh, Ir, Ni, Pd, Pt, Übergangsmetalle der 1. und 2. Nebengruppe wie Cu, Ag, Zn, seltende Erdmetalle wie La, Ce, Pr, Nd, Fr, Yb und U eingesetzt. Vorteilhaft beträgt der Gehalt an den genannten Metallen 0,1 bis 5 Gew.%.

Zweckmäßigerweise führt man die Dotierung so durch, daß man z.B. den verformten Zeolithen in einem Steigrohr vorlegt und bei 20 bis 100°C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Co(NO_3)_2$ x 3 $H_2O$ oder $Ni(NO_3)_2$ x 6 $H_2O$ oder $Ce(NO_3)_2$ x 6 $H_2O$ oder $La(NO_3)_2$ x 6 $H_2O$ oder $Ca(OH)_2$ oder $Cs_2CO_3$ in Wasser löst. Mit dieser Lösung wird der verformte oder unverformte Zeolith eine gewisse Zeit, ca. 30 Minuten, getränkt. Die eventuell überstehende Lösung wird am Rotationsverdampfer von Wasser befreit. Danach wird der getränkte Zeolith bei ca. 150°C getrocknet und bei ca. 550°C calciniert. dieser Tränkvorgang kann mehrmals hintereinander vorgenommen werden, um den gewünschten Metallgehalt einzustellen.

Auch ist es möglich, z.B. eine wäßrige $Ni(NO_3)_2$-Lösung oder ammoniakalische $Pd(NO_3)_2$-Lösung herzustellen und darin den reinen pulverförmigen Zeolithen bei 40 bis 100°C unter Rühren ca. 24 h aufzuschlämmen. Nach Abfiltrieren, Trocknen bei ca. 150°C und Calcinierung bei ca. 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige $Ni(NO_3)_2$-Lösung oder ammoniakalische $Pd(NO_3)_2$-Lösung bei leicht erhöh-ter Temperatur zwischen 30 und 80°C im Kreislauf 15 bis 20 h leitet. Danach wird mit Wasser ausgewaschen, bei ca. 150°C getrocknet und bei ca. 550°C calciniert. Bei manchen metalldotierten Zeolithen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft z.B. so vor, daß man Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert.

Nach einer anderen Arbeitsweise behandelt man Zeolithe vor oder nach ihrer Verformung mit Bindemitteln, z.B. 1 bis 3 Stunden bei Temperaturen von 60 bis 80°C mit einer 3 bis 25 gew.%igen, insbesondere 12 bis 20 gew.%igen wäßrigen Salzsäure. Anschließend wird der so behandelte Zeolith mit Wasser gewaschen, getrocknet und bei 400°C bis 500°C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen, des zeolithischen Materials wird dieses zweckmäßig, z.B. bei Temperaturen von 100 bis 160°C, getrocknet und bei Temperaturen von im allgemeinen 450°C bis 600°C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50°C bis 90°C, vorzugsweise 60°C bis 80°C, über einen Zeitraum von 0,5 bis 5, vorzugsweise mit 12 bis 20 gew.%iger Salzsäure, behandelt. Anschließend wird das zeolithische Material im allgemeinen ausgewaschen und zweckmäßig, z.B. bei Temperaturen von 100 bis 160°C, getrocknet und bei Temperaturen von im allgemeinen 450 bis 600°C calciniert. Einer HF-Behandlung kann sich auch eine HCl-Behandlung anschließen.

Die hier beschriebenen Katalysatoren können wahlweise z.B. als 2- bis 4-mm-Stränge oder als Tabletten z.B. mit 3 bis 5 mm Durchmesser oder als Splitt z.b. mit Teilchengrößen von 0,1 bis 0,5 mm oder als Wirbelkontakt eingesetzt werden.

Für das erfindungsgemäße Verfahren werden vorzugsweise folgende Reaktionsbedingungen gewählt: Das Molverhältnis Pentensäureester zu Alkanolen beträagt vorteilhaft von 1 : 0,1 bis 1 : 10, besonders 1 : 0,2 bis 1 : 3.

Die Isomerisierung führt man vorteilhaft bei einer Temperatur von 50 bis 300°C durch. Hierbei hält man in der Regel Normaldruck, Unterdruck oder auch erhöhten Druck, z.B. bix zu 50 bar ein.

In einer diskontinuierlichen Arbeitsweise wird beispielsweise in einem Rührbehälter pulverförmiger Zeolith im isomeren Pentensäure-Alkanol-Gemisch suspendiert und bei einer Temperatur von 50 bis 250°C, insbesondere 100 bis 200°C für einen Zeitraum von 2 bis 20 Stunden gerührt. Anschließend wird nach Abtrennen des Katalysators 4-Pentensäuremethylester durch Destillation isoliert.

In einer bevorzugten, kontinuierlichen Arbeitsweise wird in einem Reaktionsrohr verformter Zeolith-Katalysator vorgelegt und die zu isomerisierende Mischung aus isomeren Pentensäureestern und Alkanolen mit einer mittleren Verweilzeit von 5 bis 100 Minuten bei einer Temperatur von 50 bis 250°C, insbesondere 100 bis 200°C, vorzugsweise 100 bis 150°C, flüssig durch das Rohr gepumpt. Anschließend wird das Reaktionsgemisch nach üblichen Methoden, z.B. destillativ getrennt und nicht umgesetzte Ausgangsstoffe vorteilhaft weider zurückgeführt.

In einer besonders bevorzugten Ausführungsform des erfindungsgemäßen Verfahrens leitet man die isomeren Pentensäureester und Alkanole gasförmig gegebenenfalls zusammen mit einem inerten Trägergas wie Stickstoff über die Zeolith-Katalysatoren. Hierbei hält man vorteilhaft eine Temperatur von 150 bis 300°C, insbesondere 150 bis 250°C ein. Es hat sich bewährt, eine Belastung WHSV (g Einsatzgemisch je g Katalysator und Stunde) von 0,1 bis 20 $h^{-1}$, insbesondere 0,5 bis 5 $h^{-1}$, einzuhalten. Das erhaltene gasförmige Reaktionsgemisch wird kondensiert und 4-Pentensäuremethylester durch Destillation gewonnen.

Nach dem erfindungsgemäßen Verfahren erhältliche 4-Pentensäureester eignen sich zur Herstellung von 5-Formylvaleriansäureestern, einem Vorprodukt für die Herstellung von ε-Caprolactam, Hexandiol oder Adipinsäure.

Das Verfahren sei an folgenden Beispielen veranschaulicht.

Beispiele 1 - 5

Die Reaktionen werden unter isothermen Bedingungen in einem Rohrreaktor (Wendel 0,6 cm, 90 cm Länge) in der Gasphase mindestens 6 Stunden lang durchgeführt. Die Trennung und Charakterisierung der Reaktionsprodukte erfolgte nach üblichen Methoden. Die quantitative Bestimmung der Reaktionsprodukte und der Ausgangsstoffe erfolgte gaschromatographisch nach üblichen Methoden. Reaktionsbedingungen und Ergebnisse sind in Tabelle 1 aufgeführt.

Die in den Beispielen für das erfindungsgemäße Verfahren verwendeten Katalysatoren sind:

Katalysator A

Der Borosilikatzeolith des Pentasil-Typs wird in einer hydrothermalen Synthese aus 640 g hochdisper-sem $SiO_2$, 122 g $H_3BO_3$, 8 kg einer wäßrigen 1,6-Hexandiamin-Lösung (Mischung 50 : 50 Gew.%) bei 170°C unter autogenem Druck in einem Rührautoklaven hergestellt. Nach Abfiltrieren und Auswaschen wird das kristalline Reaktionsprodukt bei 100°C/24 h getrocknet und bei 500°C/24 h calciniert. dieser borosilikatzeolith setzt sich zusammen aus 94,2 Gew.% $SiO_2$ und 2,3 Gew.% $B_2O_3$.

Mit diesem Material werden durch Verformen mit Verformungshilfsmitteln 2-mm-Stränge hergestellt, die bei 110°C/16 h getrocknet und bei 500°C/24 h calciniert werden.

Katalysator A wird durch Imprägnieren von obigen Strängen mit einer $Pd(NO_3)_2$/$NaNO_3$-Lösung erhalten. Nach Trocknung bei 130°C/2 h und Calcination bei 540°C/2 h beträgt der Pd-Gehalt 2,5 Gew.% und der Na-Gehalt 1,0 Gew.%.

Katalysator B

Katalysator B wird wie Katalysator A erhalten, jedoch beträgt der Pd-Gehalt 0,5 Gew.% und der Na-Gehalt 1,0 Gew.%.

Katalysator C

Kommerziell erhältlicher Na-Y-Zeolith wird mit wäßriger $(NH_4)_2SO_4$-Lösung nach bekannter Methode ionenausgetauscht, so lange bis der Na-Gehalt kleiner 0,05 Gew.% liegt (nach Trocknung 110°C/2 h und Calcination bei 570°C/3 h). Das so erhaltene Pulver wird mit Verformungshilfsmitteln zu Strängen verformt, bei 110°C getrocknet und bei 500°C/16 h calciniert.

Katalysator D

Kommerziell erhältlicher Na-Y-Zeolith wird mit Boehmit im Gewichtsverhältnis 60 : 40 verstrangt, bei 110°C getrocknet und bei 500°C/16 h calciniert und mit 20%iger Ammoniumchloridlösung einem Ionenaustausch unterworfen. Der Restnatriumgehalt beträgt 0,2 Gew.% (500°C calciniert).

Diese Stränge werden mit wäßriger $Co(No_3)_2$-Lösung nach bekannter Methode imprägniert. Nach Trocknung bei 110°C und Calcination bei 540°C/2 h beträgt der Co-Gehalt 1,5 Gew.%.

Katalysator E

Katalysator E wird erhalten, indem man Katalysator C, wie bei Katalysator A beschrieben, mit wäßriger $Pd(NO_3)_2$-Lösung imprägniert. Der Pd-Gehalt beträgt 0,4 Gew.%.

Die mit obigen Katalysatoren erzielten Ergebnisse und die Reaktionsbedingungen sind in Tabelle 1 angegeben.

Tabelle 1: Isomerisierung von 3-Pentensäuremethylester (PSE)/CH₃OH

| Beispiel | 1 | 2 | 3 | 4 | 5 |
|---|---|---|---|---|---|
| 3-PSE/CH₃OH molar | 1:0,5 | 1:0,5 | 1:0,75 | 1:0,5 | 1:0,75 |
| Katalysator | A | B | C | D | E |
| Temperatur | 180°C | 180°C | 180°C | 180°C | 180°C |
| WHSV | 1,3 h⁻¹ | 1,3 h⁻¹ | 1,3 h⁻¹ | 1,3 h⁻¹ | 1,3 h⁻¹ |

Flüssiger Austrag enthält in Fl.-%

| | | | | | |
|---|---|---|---|---|---|
| 4-PSE | 7,7 | 4,8 | 7,8 | 7,0 | 9,2 |
| 3-PSE | 69,3 | 89,3 | 66,3 | 80,0 | 62,8 |
| 2-PSE | 7,5 | 3,9 | 6,0 | 3,1 | 13,2 |

Beispiele 6 - 11

In den Beispielen 6 - 11 wird gezeigt, daß die Zugabe von Methanol zu dem eingesetzten Pentensäuremethylester auf die Laufzeit des katalysators eine deutlichere Verbesserung erbringt, als die Zugaben anderer organischer Lösungmittel. Diese Versuche wurden an Katalysator F ausgeführt. Reaktionsbedingungen und Ergebnisse sind in Tabelle 2 aufgeführt.

Katalysator F

Katalysator F wird erhalten, indem man den bei Katalysator A hergestellten Borosilikatzeolith mit Boehmit im Gewichtsverhältnis 60 : 40 zu 2-mm-Strängen verformt, bei 130°C trocknet und bei 500°C/16 h calciniert. Diese Stränge werden mit einer wäßrigen $Pd(NO_3)_2/Ca(NO_3)_2$-Lösung imprägniert nach

bekannter Methode. Nach abermaliger Trocknung bei 130° C und Calcination bei 540° C/2 h beträgt der Pd-Gehalt 1,3 Gew.% und der Ca-Gehalt 0,3 Gew.%.

Die Versuchsergebnisse und Versuchsbedingungen sind in Tabelle 2 niedergelegt.

Tabelle 2

| Beispiel | 6 | 7 | 8 Vergleich | 9 Vergleich | 10 Vergleich | 11 Vergleich |
|---|---|---|---|---|---|---|
| LM[1] | $CH_3OH$ | $CH_3OH$ | Toluol | Toluol | THF | THF |
| 3-PSE/LM[2] | 1:1 | 1:1 | 1:0,3 | 1:0,3 | 1:0,5 | 1:0,5 |
| Temperatur | 180° C | 180° C | 180° C | 180° C | 180° C | 180° C |
| WHSV | $0,4\ h^{-1}$ | $0,4\ h^{-1}$ | $0,4\ h^{-1}$ | $0,4\ h^{-1}$ | $0,4\ h^{-1}$ | $0,4\ h^{-1}$ |
| Zeit[3] | 26 h | 102 h | 26 h | 102 h | 26 h | 102 h |

Der flüssige Austrag enthält in Fl.-%

| | | | | | | |
|---|---|---|---|---|---|---|
| 4-PSE | 7,3 | 8,3 | 3,9 | 1,8 | 3,3 | 1,6 |
| 3-PSE | 55,3 | 67,8 | 78,3 | 94,4 | 92,7 | 93,4 |
| 2-PSE | 25,5 | 17,4 | 3,9 | 2,1 | 2,3 | 1,4 |

1) LM = Lösungsmittel

2) molares Verhältnis

3) Zeit der Probenahme

Beispiele 12 - 14

In den Beispielen 12 - 14 wird der standverbessernde Einfluß durch Methanolzusatz veranschaulicht. Hierbei wird Katalysator G eingesetzt.

Katalysator G

Die bei Katalysator A beschriebenen Stränge des Borosilikatzeolithen werden in einem Steigrohr vorgelegt und mit einer 20%igen wäßrigen $NH_4Cl$-Lösung bei 80° C/2 h ionenausgetauscht. Danach wird eine ammoniakalische $Pd(NO_3)_2$-Lösung (11 Gew.% Pd) übergeleitet. nach Trocknung bei 110° C und Calcination bei 500° C/5 h beträgt der Pd-Gehalt 2 Gew.%.

Die erzielten Ergebnisse und Versuchsparameter sind in Tabelle 3 zusammengestellt.

Tabelle 3: Isomerisierung von 3-Pentensäuremethylester
(3-PSE)/CH$_3$OH Gemischen

| Beispiel | 12 | | | 13 | | | | 14 | | | |
|---|---|---|---|---|---|---|---|---|---|---|---|
| 3-PSE/LM[1] | 1:0 | | | 1:1 | | | | 1:2 | | | |
| Temperatur | 180°C | | | 180°C | | | | 180°C | | | |
| WHSV | 1,3 h$^{-1}$ | | | 1,3 h$^{-1}$ | | | | 1,3 h$^{-1}$ | | | |
| Zeit | 3 h | 11 h | 40 h | 3 h | 11 h | 40 h | 120 h | 3 h | 11 h | 40 h | 120 h |

PSE-Gehalt im flüssigen Austrag in Fl.-%

| 4-PSE | 10,0 | 4,4 | 1,4 | 10,0 | 9,8 | 6,8 | 5,0 | 9,1 | 8,8 | 9,0 | 7,6 |

1) molares Verhältnis

## Patentansprüche

1. Verfahren zur Herstellung von 4-Pentensäureestern durch Isomerisierung, wobei man isomere Pentensäureester bei erhöhter Temperatur mit Zeolithen behandelt und 4-Pentensäureester aus dem Reaktionsgemisch abdestilliert, dadurch gekennzeichnet, daß man Pentensäureester im Gemisch mit Alkanolen einsetzt.

2. Verfahren nach Anspruch 1, dadurch gekennzeichnet, daß das Molverhältnis von Pentensäureester zu Alkanol 1 : 0,1 bis 10 beträgt.

3. Verfahren nach den Ansprüchen 1 und 2, dadurch gekennzeichnet, daß man Alkanole mit bis zu 3 Kohlenstoffatomen oder deren Gemische einsetzt.

4. Verfahren nach den Ansprüchen 1 bis 3, dadurch gekennzeichnet, daß man als Katalysatoren Zeolithe des Pentasiltyps verwendet.

5. Verfahren nach den Ansprüchen 1 bis 3, dadurch gekennzeichnet, daß man als Katalysatoren Zeolithe des Faujasittyps verwendet.

6. Verfahren nach den Ansprüchen 1 bis 5, dadurch gekennzeichnet, daß man mit Edelmetallen und/oder Übergangsmetallen dotierte Zeolithe verwendet.

## Claims

1. A process for preparing a 4-pentenoic ester by isomerizing an isomeric pentenoic ester by treatment with a zeolite at elevated temperatures and removal of the 4-pentenoic ester from the reaction mixture by distillation, which comprises using the pentenoic ester in a mixture with an alkanol.

2. A process as claimed in claim 1, wherein the molar ratio of pentenoic ester:alkanol is 1:0.1 to 10.

3. A process as claimed in claims 1 and 2, wherein alkanols having up to 3 carbon atoms or mixtures thereof are used.

4. A process as claimed in claim 1 or 2 or 3, wherein the catalyst used is a zeolite of the pentasil type.

5. A process as claimed in claim 1 or 2 or 3, wherein the catalyst used is a zeolite of the faujasite type.

6. A process as claimed in claim 1 or 2 or 3 or 4 or 5, wherein the zeolite used has been doped with a noble metal or a transition metal.

## Revendications

1. Procédé de préparation d'esters de l'acide 4-penténoïque par isomérisation, dans lequel on traite des esters isomères de l'acide penténoïque à haute température avec des zéolites et, par distillation, on sépare les esters de l'acide 4-penténoïque du mélange réactionnel, caractérisé en ce qu'on utilise les esters de l'acide penténoïque en mélange avec des alcanols.

2. Procédé selon la revendication 1, caractérisé en ce que le rapport en moles des esters de l'acide penténoïque à l'alcanol est de 1:0,1 à 10.

3. Procédé selon les revendications 1 et 2, caractérisé en ce qu'on utilise des alcanols ayant jusqu'à 3 atomes de carbone, ou leurs mélanges.

4. Procédé selon les revendications 1 à 3, caractérisé en ce qu'on utilise comme catalyseurs des zéolites du type pentasil.

5. Procédé selon les revendications 1 à 3, caractérisé en ce qu'on utilise comme catalyseurs des zéolites du type faujasite.

6. Procédé selon les revendications 1 à 5, caractérisé en ce qu'on utilise des zéolites dopées par des métaux précieux et/ou des métaux de transition.